# EUROPEAN PATENT APPLICATION

(11) **EP 4 843 613 A1**
(43) Date of publication of application: **25.11.2026**
(21) Application number: 25177303.2
(22) Date of filing: 19.05.2025
(51) Int. Cl.: A61N 1/39, A61N 1/04

(54) **EXTERNAL DEFIBRILLATOR CHEST PAD**

(71) Applicant: Everest Acquisition Entity, LLC, Wilmington, DE 19805 (US)
(72) Inventor: JAMODKAR, Abhijeet Dnyaneshwar, 5656 AG Eindhoven (NL); PANDEY, Alok Vishambhar Dayal, 5656 AG Eindhoven (NL); TASKAR, Vaishnavi, 5656 AG Eindhoven (NL); SOLANKURE, Ajit Ashok, 5656 AG Eindhoven (NL)
(74) Representative: Weilnau, Carsten

(57) **Abstract**

An external defibrillation chest pad (40) employing a defibrillation controller (50), a defibrillation shock module (60) and a pair of defibrillation electrode pads (70). When the external defibrillation chest pad (40) is adhered to a chest of a patient, when the external defibrillation chest pad (40) is in data communication with a hand-held device (80) and when the external defibrillation chest pad (40) is in power communication with the hand-held device (80) and/or a power bank (90), the pair of defibrillation electrode pads (70) conduct electric cardiac signals of the patient to the defibrillation controller (50), which analyzes an electrocardiogram of the patient derived from the electric cardiac signals. The defibrillation controller (50) further communicates the electrocardiogram of the patient to the hand-held device (80), and controls a cardiac therapy from the defibrillation shock module (60) through the pair of defibrillation electrode pads (70) to the patient based on the analysis of the electrocardiogram of the patient indicating a shockable rhythm within the electrocardiogram of the patient.

## Description

### FIELD OF THE INVENTION

The present disclosure generally relates to external defibrillators. The present disclosure particularly relates to a novel and unique version of external defibrillators.

### BACKGROUND OF THE INVENTION

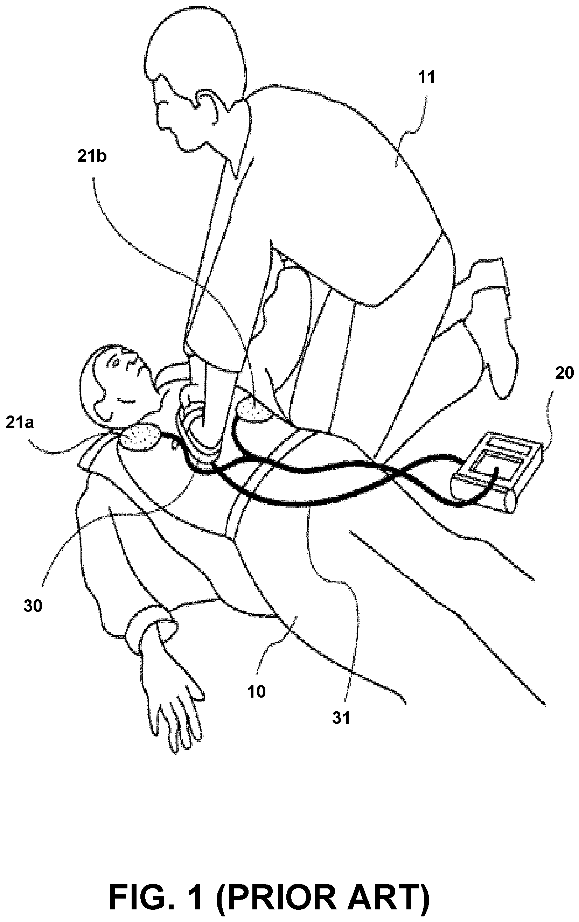

As shown in FIG. 1, an external defibrillator 20 (e.g., automatic, semi-automatic or manual) is attached across a chest of patient 10 by a pair of defibrillation electrode pads 21a and 21b to acquire electrical cardiac signals of patient 10.

In an automatic version of external defibrillator 20 as known in the art of the present disclosure, external defibrillator 20 analyzes an electrocardiogram derived from the electrical cardiac signals of patient 10 to ascertain if patient 10 is experiencing an arrhythmia (e.g., ventricular fibrillation (VF) or ventricular tachycardia (VT)) that is not accompanied by spontaneous circulation) and automatically delivers a high-voltage impulse to the heart of patient 10 via defibrillation electrode pads 21a and 21b in an attempt to restore a normal rhythm and contractile function for the heart of patient 10.

In a semi-automatic version of external defibrillator 20 as known in the art of the present disclosure, external defibrillator 20 analyzes an electrocardiogram derived from the electrical cardiac signals of patient 10 to ascertain if patient 10 is experiencing an arrhythmia (e.g., ventricular fibrillation (VF) or ventricular tachycardia (VT)) that is not accompanied by spontaneous circulation), and prompts a responder 11 to activate a delivery of a high-voltage impulse to the heart of patient 10 via defibrillation electrode pads 21a and 21b in an attempt a restore of normal rhythm and contractile function for the heart of patient 10.

Also as shown in FIG. 1, a CPR coaching device 30 may be coupled to defibrillator 20 by an electric cable 31 to provide defibrillator 20 with physiological information obtained by sensors contained in the CPR coaching device 30 as known in the art of the present disclosure. Electric cable 31 provides power for electronic components in CPR coaching device 30 and couples compression related signals to defibrillator 20 (e.g., compression depth, compression rate, chest release and recoil) whereby the physiological information indicated by the signals can be used by defibrillator 20 to issue audible CPR instructions through the loudspeaker of defibrillator 20.

The defibrillator industry is constantly striving to improve upon existing versions of external defibrillators and to uniquely design novel versions of external defibrillators.

Additionally, defibrillation electrode pads (e.g., pads 21a and 21b) are essential to establishing a proper electrical connection between a patient and an external defibrillator.

The defibrillator industry is also constantly striving to improve upon existing electrical connectors for establishing a proper electrical connection between a patient and an external defibrillator.

### SUMMARY OF THE INVENTION

The present disclosure provides an external defibrillation chest pad as an alternative to automatic external defibrillators and semi-automatic external defibrillators as known in the art of the present disclosure.

The present disclosure can be embodied as (1) an external defibrillation chest pad, (2) an external defibrillation system utilizing an external defibrillation chest pad, and (3) an external defibrillation method executable by an external defibrillation chest pad.

Various embodiments of an external defibrillation chest pad of the present disclosure encompass the external defibrillation chest pad housing a defibrillation controller, a defibrillation shock module and a pair of defibrillation electrode pads.

The pair of defibrillation electrode pads can be detachably housed within the external defibrillation chest pad by a spade connector.

In a pre-operation phase, the external defibrillation chest pad is adhered to a chest of a patient, a data communication is established between the external defibrillation chest pad and a hand-held device (e.g., a smartphone or a tablet), and a power communication is established between the external defibrillation chest pad and the hand-held device and/or a power bank.

In an operation phase, the defibrillation electrode pads are configured to conduct electric cardiac signals of the patient to the defibrillation controller, and the defibrillation controller is configured to (1) analyze an electrocardiogram of the patient derived from the electrical cardiac signals conducted by the pair of defibrillation electrode pads to the defibrillation controller, (2) communicate the electrocardiogram of the patient to the hand-held device, and (3) control a cardiac therapy from the defibrillation shock module through the pair of defibrillation electrode pads to the patient based on the analysis of the electrocardiogram of the patient indicating a shockable cardiac rhythm of the patient.

The control of the cardiac therapy can be executed by the defibrillation controller in an automatic external defibrillation mode, by the defibrillation controller in conjunction with the hand-held device in a semi-automatic external defibrillation mode, or by the defibrillation controller in conjunction with the hand-held device in a manual defibrillation mode.

Various embodiments of an external defibrillation chest pad of the present disclosure can further encompass the defibrillator controller configured to control a (4) charging of the defibrillation shock module by the hand-held device and/or a power bank.

Various embodiments of a defibrillation controller of the external defibrillation chest pad of the present disclosure employ a non-transitory machine-readable storage medium encoded with instructions for execution by one or more processors to (1) analyze an electrocardiogram of the patient derived from electrical cardiac signals conducted by a pair of defibrillation electrode pads to the defibrillation controller, (2) communicate the electrocardiogram of the patient to a hand-held device, and (3) control a cardiac therapy from the defibrillation shock module through the pair of defibrillation electrode pads to the patient based on the analysis of the electrocardiogram of the patient indicating a shockable cardiac rhythm of the patient.

The non-transitory machine-readable storage medium can be further encoded with instructions for execution by the processor(s) to (4) control a charging of the defibrillation shock module by the hand-held device and/or a power bank when the external defibrillation chest pad is in power communication with the hand-held device and/or the power bank.

Various embodiments of an external defibrillation system of the present disclosure encompasses a handheld device, and an external defibrillation chest pad housing a defibrillation controller, a defibrillation shock module and a pair of defibrillation electrode pads.

The pair of defibrillation electrode pads can be detachably housed within the external defibrillation chest pad by a spade connector.

**In** a pre-operation phase, the external defibrillation chest pad is adhered to a chest of a patient, a data communication is established between the external defibrillation chest pad and a hand-held device (e.g., a smartphone or a tablet), and a power communication is established between the external defibrillation chest pad and the hand-held device and/or a power bank.

**In** an operation phase, the defibrillation electrode pads are configured to conduct electric cardiac signals of the patient to the defibrillation controller, and the defibrillation controller is configured to (1) analyze an electrocardiogram of the patient derived from the electrical cardiac signals conducted by the pair of defibrillation electrode pads to the defibrillation controller, (2) communicate the electrocardiogram of the patient to the hand-held device, and (3) control a cardiac therapy from the defibrillation shock module through the pair of defibrillation electrode pads to the patient based on the analysis of the electrocardiogram of the patient indicating a shockable cardiac rhythm of the patient.

The control of the cardiac therapy can be executed by the defibrillation controller in an automatic external defibrillation mode, by the defibrillation controller in conjunction with the hand-held device in a semi-automatic external defibrillation mode, or by the defibrillation controller in conjunction with the hand-held device in a manual defibrillation mode.

Various embodiments of an external defibrillation system of the present disclosure can further encompass the defibrillator controller configured to (4) control a charging of the defibrillation shock module by the hand-held device and/or the power bank.

Various embodiments of an external defibrillation system of the present disclosure can further encompass the hand-held device configured to generate a display of a user interface analogous to user interface displayed on external defibrillators as known in the art of the present disclosure (e.g., an interactive display of an electrocardiogram of the patient and information corresponding to a shock advisory and any shock delivery, and an interactive selection or streaming of training/setup videos), and/or the hand-held device communicating important information to the responder (e.g., voice recordings and warning tones to prompt the responder for specific actions/information, particularly related to shock delivery and/or a CPR application).

Various embodiments of an external defibrillation system of the present disclosure can further encompass the hand-held device configured to generate a user interface analogous to user interface displayed on external defibrillators as known in the art of the present disclosure (e.g., an interactive display of an electrocardiogram of the patient and information corresponding to a shock advisory and any shock delivery, and an interactive selection or streaming of training/setup videos), and/or the hand-held device communicating important information to the responder (e.g., voice recordings and warning tones to prompt the responder for specific actions/information, particularly related to shock delivery and/or a CPR application).

Various embodiments of an external defibrillation system of the present disclosure can further encompass the hand-held device configured to communicate with remote rescue device(s)/system(s) (e.g., via cloud application, Internet and GPS connectivity) for implementing various applications including, but not limited to, centralized monitoring and tracking of external defibrillation chest pads and communication with local emergency services.

Various embodiments of an external defibrillation method of the present disclosure are executable by an external defibrillation chest pad housing a defibrillation controller, a defibrillation shock module and a pair of defibrillation electrode pads.

In a pre-operation phase, the external defibrillation method encompasses adhering the external defibrillation chest pad is adhered to a chest of a patient, establishing a data communication between the external defibrillation chest pad and a hand-held device (e.g., a smartphone or a tablet), and establishing a power communication between the external defibrillation chest pad and the hand-held device and/or a power bank.

In an operation phase, the external defibrillation method encompasses the pair of defibrillation electrode pads conducting electric cardiac signals of the patient to the defibrillation controller.

In the operation phase, the external defibrillation method further encompasses the defibrillation controller (1) analyzing an electrocardiogram of the patient derived from the electrical cardiac signals conducted by the pair of defibrillation electrode pads to the defibrillation controller, (2) communicating the electrocardiogram of the patient to the hand-held device, and (3) controlling a cardiac therapy from the defibrillation shock module through the pair of defibrillation electrode pads to the patient based on the analysis of the electrocardiogram of the patient indicating a shockable cardiac rhythm of the patient.

The controlling of the cardiac therapy can be executed by the defibrillation controller in an automatic external defibrillation mode, by the defibrillation controller in conjunction with the hand-held device in a semi-automatic external defibrillation mode, or by the defibrillation controller in conjunction with the hand-held device in a manual defibrillation mode.

Various embodiments of an external defibrillation method of the present disclosure can further encompass the defibrillator controller (4) controlling a charging of the defibrillation shock module by the hand-held device and/or the power bank.

Various embodiments of an external defibrillation method of the present disclosure can further encompass the hand-held device generating a user interface analogous to user interface displayed on external defibrillators as known in the art of the present disclosure (e.g., an interactive display of an electrocardiogram of the patient and information corresponding to a shock advisory and any shock delivery, and an interactive selection or streaming of training/setup videos), and/or the hand-held device communicating important information to the responder (e.g., voice recordings and warning tones to prompt the responder for specific actions/information, particularly related to shock delivery and/or a CPR application).

Various embodiments of an external defibrillation method of the present disclosure can further encompass the hand-held device generating a user interface analogous to user interface displayed on external defibrillators as known in the art of the present disclosure (e.g., an interactive display of an electrocardiogram of the patient and information corresponding to a shock advisory and any shock delivery, and an interactive selection or streaming of training/setup videos), and/or the hand-held device communicating important information to the responder (e.g., voice recordings and warning tones to prompt the responder for specific actions/information, particularly related to shock delivery and/or a CPR application).

Various embodiments of an external defibrillation method of the present disclosure can further encompass the hand-held device communicating with remote rescue device(s)/system(s) (e.g., via cloud application, Internet and GPS connectivity) for implementing various applications including, but not limited to, centralized monitoring and tracking of external defibrillation chest pads and communication with local emergency services.

The foregoing exemplary embodiments and other embodiments of the present disclosure as well as various structures and advantages of the present disclosure will become further apparent to those having ordinary skill in the art from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments with reference to the following figures wherein:
FIG. 1 illustrates a rescue attempt being administered by a responder to a heart of a patient as known in the art of the present disclosure;
FIG. 2 illustrates a rescue attempt being administered by a responder to a heart of a patient in accordance with the present disclosure;
FIG. 3 illustrates a first exemplary embodiment of an external defibrillation system in accordance with the present disclosure;
FIG. 4 illustrates a flowchart representative of an exemplary embodiment of a defibrillation treatment method in accordance with the present disclosure;
FIG. 5 illustrates a flowchart representative of an exemplary embodiment of rescue method in accordance with the present disclosure;
FIG. 6 illustrates an exemplary embodiment of an external defibrillation chest pad of the present disclosure as illustrated in FIG. 1 of the present disclosure;
FIG. 7 illustrates an exemplary embodiment of an external defibrillation chest pad of the present disclosure as illustrated in FIG. 6 of the present disclosure;
FIG. 8 illustrates an exemplary embodiment of a controller of an external defibrillation pad of the present disclosure;
FIG. 9 illustrates a second exemplary embodiment of an external defibrillation system in accordance with the present disclosure;
FIG. 10 illustrates a defibrillation pad assembly as known in the art of the present disclosure;
FIG. 11 illustrates an exemplary embodiment of a defibrillation pad assembly in accordance with the present disclosure; and
FIG. 12 illustrates an exemplary embodiment of a spade connector in accordance with the present disclosure.

### DETAILED DESCRIPTION OF THE PREFERRED EMBODIMENTS

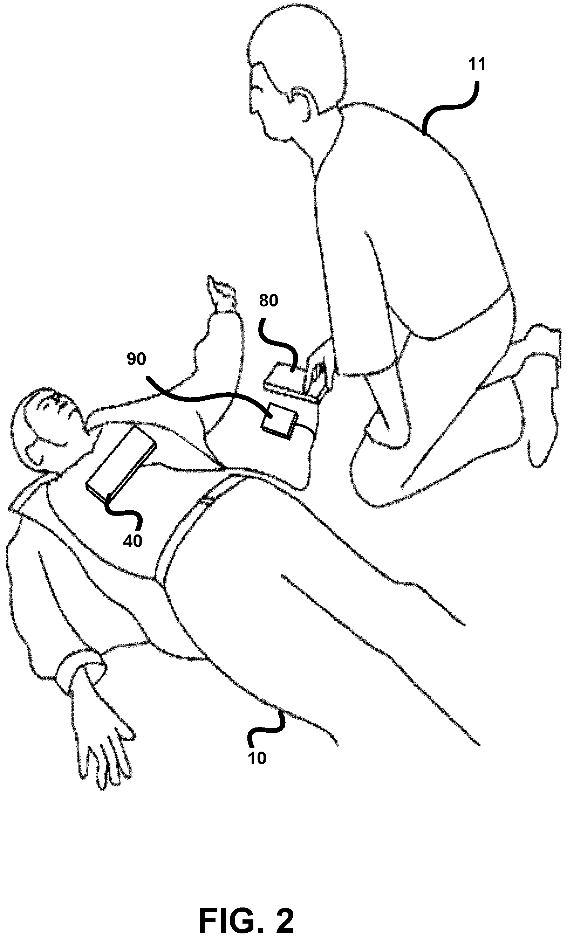

FIG. 2 illustrates an external defibrillation chest pad 40 of the present disclosure. as an alternative rescue device to external defibrillator 20 of FIG. 1. As will be further described in the present disclosure in detail, an utilization of external defibrillation chest pad 40 in a rescue attempt of a patient involves a pre-operation phase and an operation phase.

Referring to FIG. 2, in the pre-operation phase, external defibrillation chest pad 40 is adhered to a chest of a patient, a data communication is established between external defibrillation chest pad 40 and a hand-held device 80 (e.g., a smartphone or a tablet), and a power communication is established between external defibrillation chest pad 40, hand-held device 80 and/or a power bank 90.

Still referring to FIG. 2, in the operation phase, defibrillation electrode pads (not shown) of external defibrillation chest pad 40 conduct electric cardiac signals of the patient to the defibrillation controller (not shown) of external defibrillation chest pad 40, and the defibrillation controller analyzes an electrocardiogram of the patient derived from the electrical cardiac signals conducted by the pair of defibrillation electrode pads to the defibrillation controller, communicates the electrocardiogram of the patient to hand-held device 80, and controls a cardiac therapy from a defibrillation shock module (not shown) of external defibrillation chest pad 40 through the pair of defibrillation electrode pads of external defibrillation chest pad 40 to the patient based on the analysis of the electrocardiogram of the patient indicating a shockable cardiac rhythm of the patient.

The control of the cardiac therapy can be executed by the defibrillation controller of external defibrillation chest pad 40 in an automatic external defibrillation mode, by the defibrillation controller of external defibrillation chest pad 40 in conjunction with the hand-held device 80 in a semi-automatic external defibrillation mode, or by the defibrillation controller of external defibrillation chest pad 40 in conjunction with the hand-held device 80 in a manual defibrillation mode.

External defibrillation chest pad 40 can also control a charging of the defibrillation shock module External defibrillation chest pad 40 by the hand-held device 80 and/or a power bank 90.

For purposes of describing and claiming the present disclosure, terms of the art of the present disclosure, but not limited to, "defibrillation", "defibrillator", "defibrillating shock", "defibrillation electrode pad", "electrocardiogram (ECG)", "cardiopulmonary resuscitation (CPR)", "hand-held device", "power bank", "rescue protocol", "CPR protocol", and "shock protocol" are to be interpreted as known in the art of the present disclosure and as exemplary described in the present disclosure.

Also purposes of describing and claiming the present disclosure, the term "data communication" encompasses techniques for communicating a data signal from one device/system to another device/system as known in the art of the present disclosure and hereinafter conceived, and the term "power communication" encompasses techniques for communicating a power signal from one device/system to another device/system as known in the art of the present disclosure and hereinafter conceived.

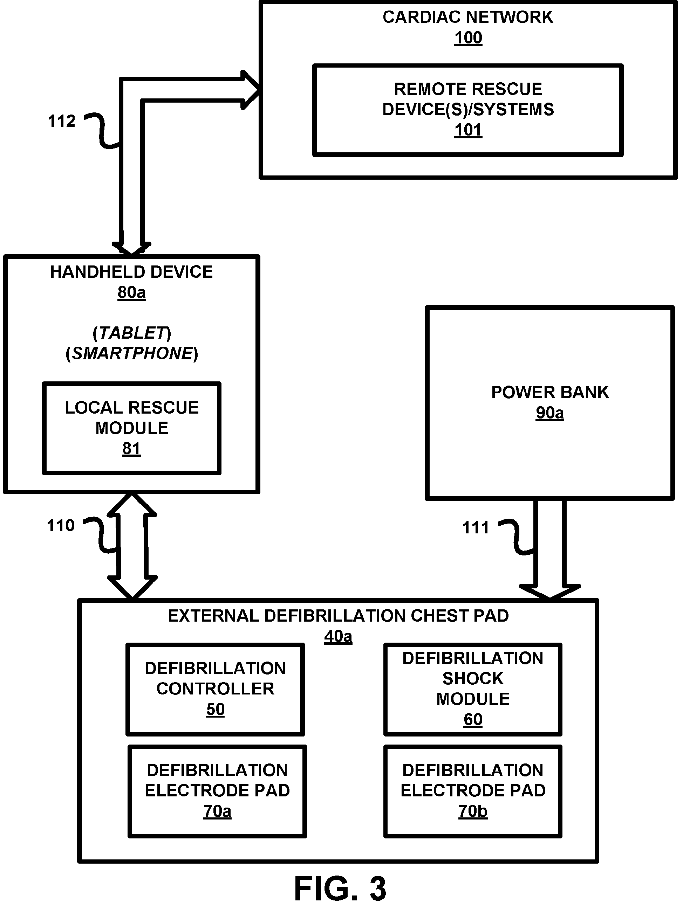

To facilitate an understanding of the present disclosure, the following description of FIGS. 3-8 describes and teaches exemplary embodiments of devices, systems and methods in accordance with the present disclosure. From the description of FIGS. 3-8, those having ordinary skill in the art of the present disclosure will appreciate how to apply the present disclosure to make and use additional embodiments of devices, systems and methods in accordance with the present disclosure.

FIG. 3 illustrates an exemplary embodiment 40a of external defibrillation chest pad 40 as shown in FIG. 2.

Referring to FIG. 3, external defibrillation chest pad 40 employs a defibrillation controller 50, a defibrillation shock module 60 and a pair of defibrillation electrode pads 70a and 70b.

Defibrillation controller 50 encompasses structural configurations, as understood in the art of the present disclosure, of an application specific main board or an application specific integrated circuit for executing cardiac analysis applications and cardiac therapy applications of the present disclosure. The structural configuration of defibrillation controller 50 can include, but is not limited to, processor(s), computer-usable/computer readable storage medium(s), an operating system, peripheral device controller(s), slot(s) and port(s).

Still referring to FIG. 3, the cardiac analysis applications incorporated within defibrillation controller 50 that can be configured as an electronic circuit (e.g., electronic components and/or hardware) and/or an executable program (e.g., executable software stored on non-transitory computer readable medium(s) and/or firmware) for implementing shock advisory methods as known in the art of the present disclosure or hereinafter conceived.

In one exemplary shock advisory embodiment of defibrillation controller 50, the cardiac analysis application implements a C-shock advisory broadly encompassing all methods, as known in the art of the present disclosure or hereinafter conceived, for analyzing and rhythm classifying an ECG of a heart of a patient including artifacts as known in the art of the present disclosure resulting from an administration of chest compressions to the heart of the patient (i.e., a corrupt ECG).

An execution of C-shock advisory renders either (1) a shock decision based upon a determinate classification of the corrupt ECG as having a shockable cardiac rhythm (e.g., a ventricular fibrillation (VF) rhythm or a ventricular tachycardia (VT) rhythm), (2) a non-shock decision based upon a determinate classification of the corrupt ECG as having a non-shockable cardiac rhythm (e.g., a pulseless electrical activity rhythm or an asystole rhythm, (3) an undecided shock decision based upon an indeterminate classification of the corrupt ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm), or (4) a terminate rescue decision based upon a determinate classification of the corrupt ECG as having a sustained cardiac rhythm (e.g., a return of an organized cardiac rhythm with or without a sustained return of a spontaneous circulation).

A non-limiting example of a C-shock advisory is an Arrythmia Recognition Technology (ART) as known in the art of the present disclosure.

In practice, the C-shock advisory, the undecided shock decision and/or the terminate rescue decision can be omitted and/or additional shock advisory decision(s) can be derived from a corrupt ECG.

In a second exemplary shock advisory embodiment of defibrillation controller 50, the cardiac analysis application implements a F-shock advisory broadly encompassing all methods, as known in the art of the present disclosure or hereinafter conceived, for analyzing and rhythm classifying an ECG of a heart of a patient excluding artifacts as known in the art of the present resulting from a termination/suspension of an administration of chest compressions to the heart of the patient (i.e., a clean ECG).

An execution of F-shock advisory renders either (1) a shock decision based upon a determinate classification of the clean ECG as having a shockable cardiac rhythm (e.g., a ventricular fibrillation (VF) rhythm or a ventricular tachycardia (VT) rhythm), (2) a non-shock based upon a determinate classification of the clean ECG as having a non-shockable cardiac rhythm (e.g., a pulseless electrical activity rhythm or an asystole rhythm), (3) an undecided shock decision based upon an indeterminate classification of the clean ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm), or (4) a terminate rescue decision based upon a determinate classification of the clean ECG as having a sustained cardiac rhythm (e.g., a return of an organized cardiac rhythm with or without a sustained return of spontaneous circulation).

A non-limiting example a F-shock advisory is a Patient Analysis System (PAS) as known in the art of the present disclosure.

In practice, the F-shock advisory, the undecided shock decision and/or the terminate rescue decision can be omitted and/or additional shock advisory decision(s) can be derived from a clean ECG.

Still referring to FIG. 3, defibrillating shock module 60 is structurally configured as known in the art of the present disclosure to deliver an electric therapy to a heart of a patient as controlled by defibrillation controller 50.

In one exemplary embodiment, defibrillating shock module 60 employs a high voltage capacitor bank (not shown) for storing a high voltage via a high voltage charger and a power supply. Defibrillating shock module 60 further employs a switching/isolation circuit (not shown) for selectively applying a specific waveform of an electric energy charge from the high voltage capacitor bank to electrode pads/paddles attached to the patient as controlled by defibrillation controller 50. In practice, the defibrillating shock may have any waveform as known in the art of the present disclosure. A non-limiting example of such a waveform is a biphasic truncated waveform.

In one exemplary shock delivery embodiment of defibrillation controller 50, the cardiac therapy application implements a C-shock delivery broadly encompassing methods, as known in the art of the present disclosure or hereinafter conceived, for delivering a defibrillating shock energy from defibrillating shock module 60 to a heart of a patient corresponding to a shock decision derived by the C-shock advisory in accordance with rules/guidelines associated with a rescue protocol (e.g., American Heart Association rules/guidelines).

In a second exemplary shock delivery embodiment of defibrillation controller 50, the cardiac therapy application implements a F-shock delivery broadly encompassing methods, as known in the art of the present disclosure or hereinafter conceived, for delivering a defibrillating shock from defibrillating shock module 60 to a heart of a patient corresponding to a shock decision derived by the F-shock advisory in accordance with rules/guidelines associated with a rescue protocol (e.g., American Heart Association rules/guidelines).

Still referring to FIG. 3, defibrillation electrode pads 70a and 70b are configured, as known in the art of the present disclosure or hereinafter conceived, for conducting electrical cardiac signals from a heart of a patient to defibrillation controller 50 and for delivering a defibrillation shock from defibrillation shock module 60 to the heart of the patient.

FIG. 3 further illustrates an exemplary embodiment 80a of hand-held device 80 (FIG. 2), an exemplary embodiment 90a of power bank 90 (FIG. 2), and an optional cardiac network 100.

Referring to FIG. 3, hand-held device 80a encompasses structural configurations, as understood in the art of the present disclosure, of any portable device designed to fit in a hand or hands and incorporates a local rescue module 81 of the present disclosure. Non-limiting examples including a medical tablet and a smartphone incorporating local rescue module 81 of the present disclosure.

The local rescue module 81 can be configured as an electronic circuit (e.g., electronic components and/or hardware) and/or an executable program (e.g., executable software stored on non-transitory computer readable medium(s) and/or firmware) for primarily or secondarily implementing a shock advisory method and/or a shock delivery method as known in the art of the present disclosure or hereinafter conceived.

In practice of the present disclosure, local rescue module 81 can be further configured and/or hand-held device 80a can further incorporate an executable application to display a user interface on hand-held device 80a analogous to user interface displayed on external defibrillators as known in the art of the present disclosure. Non-limiting examples of the user interface include an interactive display of an electrocardiogram of the patient and information corresponding to a shock advisory and any shock delivery, and an interactive selection or streaming of training/setup videos.

Further in practice of the present disclosure, local rescue module 81 can be further configured and/or hand-held device 80a can further incorporate an executable application to communicate important information between the responder and hand-held device 80a. Non-limiting examples of such communication include voice recordings and warning tones to prompt the responder for specific actions/information, particularly related to shock delivery and/or a CPR application.

Still referring to FIG. 3, power bank 90a encompasses structural configurations, as understood in the art of the present disclosure, for charging external defibrillation chest pad 40a. Non-limiting examples of power bank 90a including a portable battery pack.

In practice of the present disclosure, defibrillation controller 50 can be configured to ascertain if the current charge in an internal battery of hand-held device 80a is sufficient to execute a shock advisory and shock delivery, if applicable. In case of low charge on the internal battery of hand-held device 80a, defibrillation controller 50 can be configured to prompt the responder to connect to an external power source, such as, for example, power bank 90a.

Also in practice of the present disclosure defibrillation controller 50 can be configured to ascertain if hand-held device 80a has been utilized in a maximum number of therapies. In such a case, defibrillation controller 50 can be configured to prompt the responder to connect to an external power source, such as, for example, power bank 90a.

Cardiac network 100 encompasses an interconnected group of remote rescue devices and/or systems for generating information related to a rescue attempt of a patient in accordance with the present disclosure. Non-limiting examples of remote rescue devices and/or systems include an emergency center for generating and sharing information related to a rescue attempt of a patient in accordance with the present disclosure as will be further described in the present disclosure.

Still referring to FIG. 3, in practice, a pre-operation phase of a rescue attempt in accordance with the present disclosure involves external defibrillation chest pad 40a being adhered to a chest of a patient, a data communication 110 being established between external defibrillation chest pad 40a and a hand-held device 80a (e.g., a smartphone or a tablet), and a power communication 111 being established between external defibrillation chest pad 40a and hand-held device 80a and/or a power bank 90a.

Data communication 110 can be wired or wireless as known in the art of the present disclosure or hereinafter conceived, and power connection 111 also can be wired or wireless as known in the art of the present disclosure or hereinafter conceived.

A data communication 112 can also be established between handheld device 80a and cardiac network 100, whereby data communication 112 can be wired or wireless as known in the art of the present disclosure or hereinafter conceived.

Still referring to FIG. 3, in practice, an operation phase of a rescue attempt of the present disclosure involves defibrillation electrode pads 70a and 70b conducting electric cardiac signals of the patient to defibrillation controller 50, and in response thereto, defibrillation controller 50 analyzing an electrocardiogram of the patient derived from the electrical cardiac signals and communicates the electrocardiogram of the patient to hand-held device 80a. The operation phase further involves defibrillation controller 50 controlling a delivery of a cardiac therapy from a defibrillation shock module 60 through defibrillation electrode pads 70a and 70b to the patient based on the analysis of the electrocardiogram of the patient indicating a shockable cardiac rhythm of the patient.

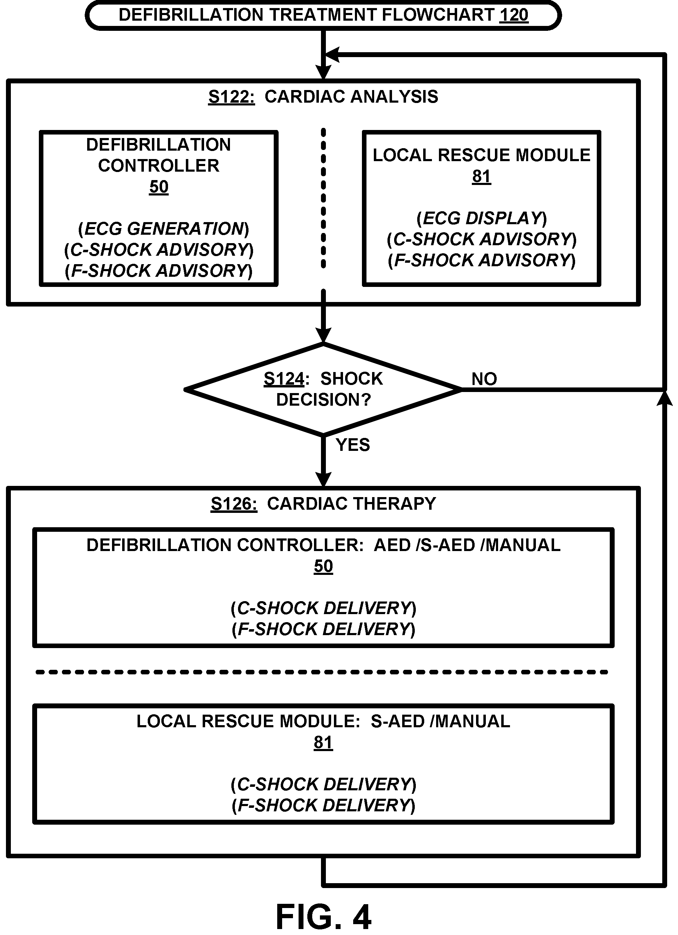

In one exemplary embodiment of the operation phase, a flowchart 120 as shown in FIG. 4 represents an exemplary embodiment of a defibrillation treatment of the present disclosure operable in an automatic mode, a semi-automatic mode, and a manual mode.

Referring to FIG. 4, in an automatic mode, a stage S122 of flowchart 120 encompasses defibrillation controller 50 implementing a cardiac analysis including a generation of an electrocardiogram of the heart of the patient and executing a C-shock advisory for analyzing and rhythm classifying a corrupt ECG of a heart of a patient as previously described in the present disclosure or a F-shock advisory for analyzing and rhythm classifying a clean ECG of a heart of a patient as previously described in the present disclosure.

Stage S122 can further include local rescue module 81 displaying the electrocardiogram for monitoring by a responder.

If the C-shock advisory or the F-shock advisory renders a shock decision during a stage S124 of flowchart 120, then flowchart 120 proceeds to a stage S126 of flowchart encompassing defibrillation controller 50 implementing a cardiac therapy including an automatic execution of a C-shock delivery to the heart of the patient as previously described in the present disclosure or a F-shock delivery to the heart of the patient as previously described in the present disclosure.

Still referring to FIG. 4, in an semi-automatic mode, stage S122 of flowchart 120 encompasses defibrillation controller 50 implementing a cardiac analysis including a generation of an electrocardiogram of the heart of the patient and executing a C-shock advisory for analyzing and rhythm classifying a corrupt ECG of a heart of a patient as previously described in the present disclosure or a F-shock advisory for analyzing and rhythm classifying a clean ECG of a heart of a patient as previously described in the present disclosure.

Stage S122 can further include local rescue module 81 displaying the electrocardiogram for monitoring by a responder.

If the C-shock advisory or the F-shock advisory renders a shock decision during a stage S124 of flowchart 120, then flowchart 120 proceeds to a stage S126 of flowchart encompassing local rescue module 81 displaying the electrocardiogram with an indication that a shock decision has been rendered. A responder can therefore interface with a key or a touchscreen of handheld device 80a to communicate a shock activation to defibrillation controller 50 and in response thereto, defibrillation controller 50 implements a cardiac therapy including an automatic execution of a C-shock delivery to the heart of the patient as previously described in the present disclosure or a F-shock delivery to the heart of the patient as previously described in the present disclosure.

Still referring to FIG. 4, in a manual mode, stage S122 of flowchart 120 encompasses defibrillation controller 50 implementing a cardiac analysis including a generation of an electrocardiogram of the heart of the patient and executing a C-shock advisory for analyzing and rhythm classifying a corrupt ECG of a heart of a patient as previously described in the present disclosure or a F-shock advisory for analyzing and rhythm classifying a clean ECG of a heart of a patient as previously described in the present disclosure.

Stage S122 includes local rescue module 81 displaying the electrocardiogram for monitoring by responder and can further includes, concurrently or alternatively, an execution of a C-shock advisory or a F-shock advisory by local rescue module 81.

If the C-shock advisory or the F-shock advisory renders a shock decision during a stage S124 of flowchart 120, then flowchart 120 proceeds to a stage S126 of flowchart encompassing local rescue module 81 displaying the electrocardiogram whereby the responder can review the displayed ECG and confirm or reject the shock decision. If the shock decision is confirmed, the responder can interface with a key or a touchscreen of handheld device 80a to communicate a shock activation to defibrillation controller 50 and in response thereto, defibrillation controller 50 implements a cardiac therapy including an automatic execution of a C-shock delivery to the heart of the patient as previously described in the present disclosure or a F-shock delivery to the heart of the patient as previously described in the present disclosure.

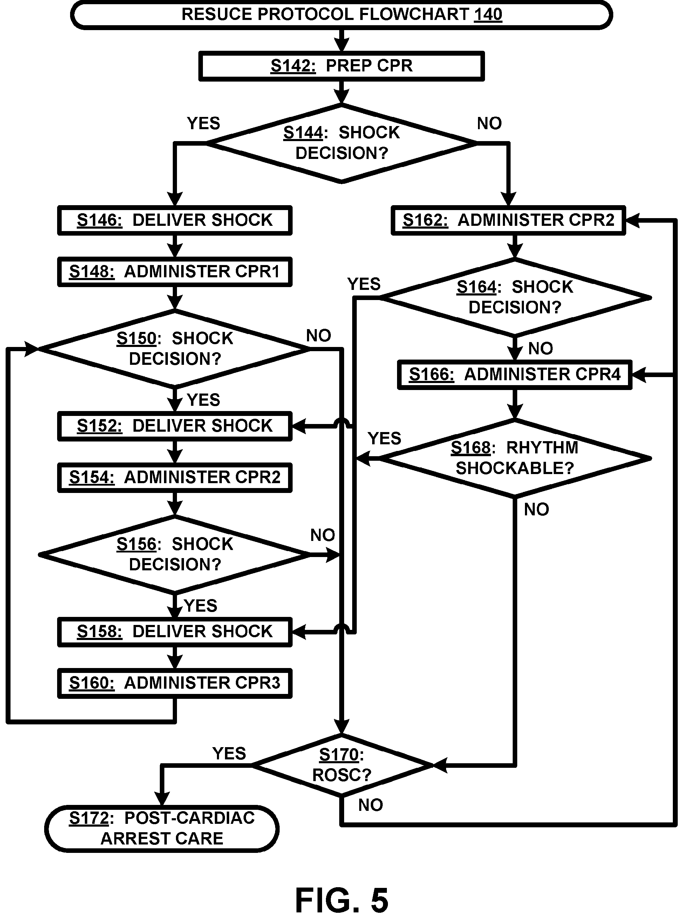

FIG. 5 depicts a flowchart 140 representative of an exemplary American Heart Association (AHA) advanced responder protocol as known in the art of the present disclosure executed in accordance with the present disclosure.

More particularly, referring to FIG. 5, a stage S142 of flowchart 140 encompasses a preparation of an administration of CPR by the responder to a patient experiencing cardiac arrest. In one embodiment of stage S142, the responder can commence providing oxygen to the patient and attach an Advanced Life Support (ALS) monitor/defibrillator to the patient.

A stage S144 of flowchart 140 encompasses a determination if a cardiac analysis in accordance with stages S122 and S124 of flowchart 120 (FIG. 4) renders a shock decision. If a shock decision is rendered during stage S 144, then a stage S 146 of flowchart 140 encompasses a delivery of a shock to the patient by external defibrillation chest pad in accordance with stage S126 of flowchart 120 (FIG. 4) and a stage S 148 of flowchart 140 encompasses an administration of a first CPR protocol to the patient.

In one embodiment of stage S 146, a biphasic shock energy of 120-200 joules can be delivered to the patient, or alternatively, a maximum available biphasic shock energy. In a second embodiment of stage S146, a monophasic shock energy of 360 joules can be delivered to the patient.

In one embodiment of stage S 148, the responder is guided by handheld device 80a (FIG. 3) to administer a quality CPR to the patient for a set time period (e.g., two (2) minutes). For example, during the set time period, the responder can be guided to push hard (e.g., 2-2.4 inches/5-6 centimeters) and fast (e.g., 140-120 compressions/minute) while allowing for a complete chest recoil. Furthermore, interruptions in compressions should be minimized, excessive ventilation should be avoided and a compressor, if applicable, should be rotated at the end of the set time period. Moreover, a 30:2 compression-ventilation ratio should be maintained if the patient does not have an advanced airway, a maximum partial pressure of CO₂ at the end a breath should be ≥ 14 mm Hg, and a relaxation phase diagnostic pressure should be ≥ 20 mm Hg. Additionally, IV/IO access to the patient can be established.

Still referring to FIG. 5, subsequent to an expiration of the set time period of stage S148, a stage S150 of flowchart 140 encompasses a determination if a cardiac analysis in accordance with stages S122 and S124 of flowchart 120 (FIG. 4) renders a shock decision. If a shock decision is rendered during stage S150 then a stage S152 of flowchart 140 encompasses a delivery of a shock to the patient by external defibrillation chest pad in accordance with stage S126 of flowchart 120 (FIG. 4) and a stage S 154 of flowchart 140 encompasses an administration of a second CPR protocol to the patient.

In one embodiment of stage S 152, a biphasic shock energy of at least 120-200 joules can be delivered to the patient, or alternatively, a maximum available biphasic shock energy. In a second embodiment of stage S152, a monophasic shock energy of 360 joules can be delivered to the patient.

In one embodiment of stage S 154, the responder is guided by handheld device 80a to administer a quality CPR to the patient as set forth in the first CPR protocol of stage S 148 for a set time period (e.g., two (2) minutes). Additionally, a Epinephrine IV/IO dose of 1mg can be delivered every 3-5 minutes to the patient by the responder and advanced airway capnography can be considered (e.g., supraglottic advanced airway or endotracheal intubation, and/or waveform capnography to confirm and monitor ET tube placement, and/or 8-10 breaths per minute with continuous chest compressions).

Still referring to FIG. 5, subsequent to an expiration of the set time period of stage S154, a stage S156 of flowchart 140 encompasses a determination if a cardiac analysis in accordance with stages S122 and S124 of flowchart 120 (FIG. 4) renders a shock decision. If a shock decision is rendered during stage S156, then a stage S158 of flowchart 140 encompasses a delivery of a shock to the patient by external defibrillation chest pad in accordance with stage S126 of flowchart 120 (FIG. 4) and a stage S 160 of flowchart 140 encompasses an administration of a third CPR protocol to the patient.

In one embodiment of stage S 158, a biphasic shock energy of at least 120-200 joules can be delivered to the patient, or alternatively, a maximum available biphasic shock energy. In a second embodiment of stage S158, a monophasic shock energy of 360 joules can be delivered to the patient.

In one embodiment of stage S 160, the responder is guided by handheld device 80a to administer a quality CPR to as set forth in the second CPR protocol the patient for a set time period (e.g., two (2) minutes). Additionally, an Amiodarone IV/IO dose of 3000 mg bolus can be delivered to the patient, and reversible causes can be treated (e.g., hypovolemia, hypoxia, hydrogen ion (acidosis), hypo-/hypercaloric, hypothermia, tension pneumothorax, cardiac tamponade, toxins, pulmonary thrombosis and coronary thrombosis).

Still referring to FIG. 5, if a cardiac analysis in accordance with stages S122 and S124 of flowchart 120 (FIG. 4) does not render a shock decision during an initial execution or iteration of stage S150 or stage S156, then a stage S170 of flowchart 140 encompasses a determination of a return of spontaneous circulation (ROSC) of the patient or no signs of ROSC of the patient. In one embodiment of stage S 170, a determination of ROSC can be derived from pulse and blood pressure of the patient, an abrupt sustained increase in a maximum partial pressure of CO₂ at the end a breath should be ≥ 40 mm Hg, and/or a spontaneous arterial wave with intra-arterial monitoring.

If a ROSC of the patient is determined during S170, then a stage S172 of flowchart 140 encompasses a post-cardiac arrest care of the patient as known in the art of the present disclosure.

If a ROSC of the patient is not determined during S170, then a stage S162 of flowchart 140 encompasses an administration of the second CPR protocol of stage S 154.

Still referring to FIG. 5, subsequent to an expiration of the set time period of stage S162, a stage S164 of flowchart 140 encompasses a determination if a cardiac analysis in accordance with stages S 122 and S 124 of flowchart 120 (FIG. 4) does not render a shock decision. If a shock decision is rendered during stage S164, then flowchart 140 proceeds to stage S152 or S158 for shock delivery as previously described. If a shock decision is not rendered during stage S164, then a stage S166 of flowchart 140 encompasses an administration of a fourth CPR protocol directed to treating reversible causes as previously described.

Still referring to FIG. 5, subsequent to an expiration of the set time period of stage S166, a stage S168 of flowchart 140 encompasses a determination if a cardiac analysis in accordance with stages S122 and S124 of flowchart 120 (FIG. 4) does not render a shock decision. If a shock decision is rendered during stage S168, then flowchart 140 proceeds to stage S152 or S158 for shock delivery as previously described. If a shock decision is not rendered during stage S168, then flowchart 140 proceeds to stage S170 for ROSC determination or non-existence as previously described.

Still referring to FIG. 5, subsequent to the CPR prep stage of S142, if a cardiac analysis in accordance with stages S122 and S124 of flowchart 120 (FIG. 4) does not render a shock decision during stage S144, then flowchart 140 proceeds to stage S162 as previously described.

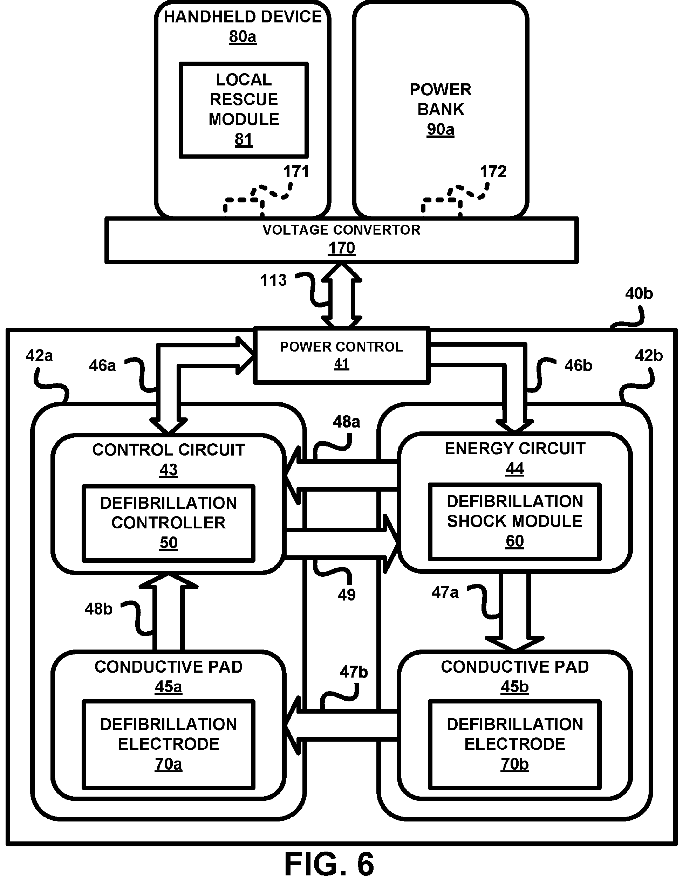

To facilitate a further understanding of the present disclosure, the following description of FIGS. 6-9 teaches exemplary embodiments of devices and systems in accordance with the present disclosure. From the description of FIGS. 6-9, those having ordinary skill in the art of the present disclosure will appreciate how to apply the present disclosure to make and use additional embodiments of devices and systems in accordance with the present disclosure.

FIG. 6 illustrates an exemplary embodiment 40b of external defibrillator chest pad 40a shown in FIG. 3.

Referring to FIG. 6, external defibrillator chest pad 40b employs a power control 41, as known in the art of the present disclosure or hereinafter conceived, in data/power connection 113 with a voltage convertor 170, as known in the art of the present disclosure or hereinafter conceived, having a USB connection 171 with handheld device 80a (FIG. 3) and a USB connection 172 with power bank 90a (FIG. 3).

External defibrillator chest pad 40b further employs a control circuit 43 primarily incorporating defibrillation controller 50 (FIG. 3), an energy circuit 44 primarily incorporating defibrillation shock module 60 (FIG. 3), a conductive pad 45a primarily incorporating defibrillation electrode 70a (FIG. 3), and a conductive pad 45b primarily incorporating defibrillation electrode 70b (FIG. 3).

A perimeter of a patient side of external defibrillation chest pad 40b has an adhesive (not shown) or adhering to a chest of a patient as known in the art of the present disclosure.

Control circuit 43 and conductive pad 45a are disposed on an apex pad 42a with conductive pad 45a having a gel, as known in the art of the present disclosure or hereinafter discovered.

Energy circuit 44 and conductive pad 45b are disposed on a sternum pad 42b with conductive pad 45b having a gel, as known in the art of the present disclosure or hereinafter discovered.

By design, control circuit 43 utilizes a data/power connection 46ato communicate data/information with handheld device 80a and to receive power from handheld device 80a and/or power bank 90a as known in the art of the present disclosure or hereinafter conceived.

Energy circuit 44 utilizes a power connection 46b to receive power from handheld device 80a and/or power bank 90a as known in the art of the present disclosure or hereinafter conceived.

Energy circuit 44 has a signal connection 48a with control circuit 43 whereby defibrillation controller 50 can monitor a charging/discharging of defibrillation shock module 60 as known in the art of the present disclosure or hereinafter conceived.

Control circuit 43 has a control connection 49 with energy circuit 44 whereby defibrillation controller 50 can control a charging/discharging of defibrillation shock module 60 as known in the art of the present disclosure or hereinafter conceived.

Energy circuit 44 has a power connections 47a and 47b with defibrillation electrodes 70a and 70b for delivering shock therapy as known in the art of the present disclosure or hereinafter conceived.

Control circuit 43 has signal connection 48a with control circuit 43 whereby defibrillation controller 50 can receive cardiac signals from defibrillation electrodes 70a and 70b as known in the art of the present disclosure or hereinafter conceived.

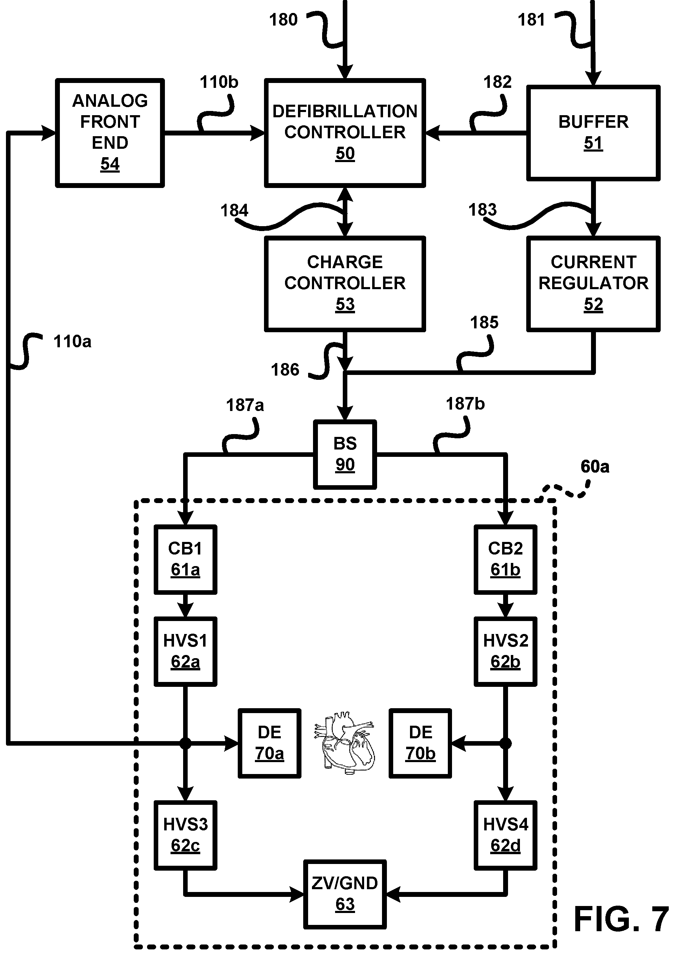

FIG. 7 illustrates an exemplary operation of an exemplary embodiment of external defibrillation chest pad 40b as shown in FIG. 6.

Referring to FIG. 7, in operation, defibrillation controller 50 receives data/power 180 from handheld device 80a and power bank 90a, and a buffer 51 receives voltage 181 (e.g., +5V) from handheld device 80a and power bank 90a. Defibrillation controller 50 receives feedback 182 from buffer 51.

Power 183 from buffer 51 is provided to a current regulator 52, which provides current regulated power 185 to a boost stage 90 as known in the art of the present disclosure or hereinafter conceived.

Defibrillation controller 50 and a charge controller 53 having a data communication 184 for generating a control signal 186 for controlling a charging current regulated power 185 of defibrillation shock module 60a via a boost stage 90 as known in the art of the present disclosure or hereinafter conceived.

Defibrillation shock module 60a includes a series connection 187a from boost stage 90 serially through a charge storage bank 61a, a high voltage switch 62a and a high voltage switch 62c to zero voltage/ground 63.

Defibrillation shock module 60a includes a series connection 187b from boost stage 90 serially through a charge storage bank 61b, a high voltage switch 62b and a high voltage switch 62d to zero voltage/ground 63.

Defibrillation electrode 70a is electrically connected between high voltage switch 62a and high voltage switch 62c, and defibrillation electrode 70b is electrically connected between high voltage switch 62b and high voltage switch 62d.

Defibrillation electrode 70a is further electrically connected to an analog front end 54 for receiving cardiac signals 110a and transmitting digitized ECG samples 110b to defibrillation controller 50 as known in the art of the present disclosure. Analog front end 54 operates to amplify, buffer, filter and optionally digitize an electrical cardiac signal 110a generated by the patient's heart to produce a stream of digitized ECG samples 110b.

When receiving electrical cardiac signals 110 from defibrillation electrodes 70a and 70b, defibrillation controller 50 will transmit switch control signals (not shown) to open high voltage switches 62a-62d while charge storage banks 61a and 61b are being charged in controlled, regulated manner, and defibrillation controller 50 executes a cardiac analysis of the digitized ECG samples 110b (e.g., stage S122 of flowchart 120 shown in FIG. 4).

Upon determining a shock decision or a shock activation from a handheld device, defibrillation controller 50 will transmit switch control signals (not shown) to close high voltage switches 62a-62d to thereby discharge charge storage banks 61a and 61b and deliver a defibrillation shock via defibrillation electrodes 70a and 70b (e.g., stages S124 and S126 of flowchart 120 shown in FIG. 4).

Subsequent to the shock delivery, defibrillation controller 50 will again transmit switch control signals (not shown) to open high voltage switches 62a-62d whereby charge storage banks 61a and 61b will be recharged in controlled, regulated manner (e.g., stage S122 of flowchart 120 shown in FIG. 4).

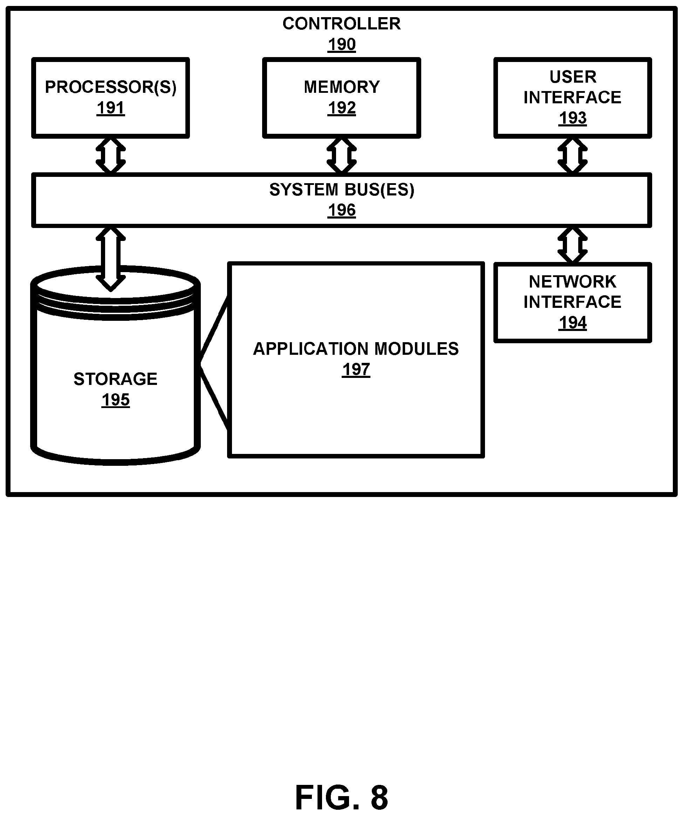

Referring to FIG. 8, shown is an exemplary embodiment of controller 190 that includes one or more processor(s) 191, memory 192, a user interface 193, a network interface 194, and a storage 195 interconnected via one or more system bus(es) 196.

Each processor 191 can be any hardware device, as known in the art of the present disclosure or hereinafter conceived, capable of executing instructions stored in memory 192 or storage or otherwise processing data. In a non-limiting example, the processor(s) 191 can include a microprocessor, field programmable gate array (FPGA), application-specific integrated circuit (ASIC), or other similar devices.

The memory 192 can include various memories, as known in the art of the present disclosure or hereinafter conceived, including, but not limited to, L1, L2, or L3 cache or system memory. In a non-limiting example, the memory 192 can include static random access memory (SRAM), dynamic RAM (DRAM), flash memory, read only memory (ROM), or other similar memory devices.

In practice, defibrillation controller 190 also provides control of the user interface (UI) output functions. Specifically, user interface 193 is the primary means for guiding the responder through the protocols of the present disclosure, and so includes at least one of an aural instruction output and a visual display. In particular, user interface 193 may comprise an audio speaker to issue an aural verbal or signal prompt to the responder regarding a state of the rescue, an instruction as to a next step to be taken in the rescue, or regarding instructions responsive to an execution of a particular protocol (e.g., administering CPR and/or delivering a drug). User interface 193 can also convey audible information via a beeper. User interface 193 can also provide visual text or graphical indications on a display. User interface 193 can also convey visual information via a flashing light LED, which may illuminate adjacent graphics or buttons to be pressed. Preferably, defibrillation controller 190 controls the user interface 191 such that each of these cues is provided in a manner that optimizes the desired response of the responder in the execution of protocols of the present disclosure.

Still referring to FIG. 8, network interface 194 can include one or more devices, as known in the art of the present disclosure or hereinafter conceived, for enabling communication with other components of defibrillator (or another device, particularly a mechanical CPR device or a CPR coaching device, as known in the art of the present disclosure or hereinafter conceived, in the administration of CPR/chest compression to a patient in accordance with the protocols of the present disclosure and/or in the acquisition of CPR data indicative of the quality of CPR being administered to the patient.

In a non-limiting example, the network interface 194 can include a network interface card (NIC) configured to communicate according to the Ethernet protocol. Additionally, the network interface 194 may implement a TCP/IP stack for communication according to the TCP/IP protocols. Various alternative or additional hardware or configurations for the network interface 194 will be apparent.

The storage 195 can include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. In various non-limiting embodiments, the storage 195 can store instructions for execution by the processor(s) 191 or data upon with the processor(s) 191 may operate. For example, the storage 195 may store a base operating system for controlling various basic operations of the hardware.

The storage 195 can also store an application modules 197 in the form of executable software/firmware for implementing the method(s) of the present disclosure as previously described in the present disclosure (e.g., flowchart 120 of FIG. 4).

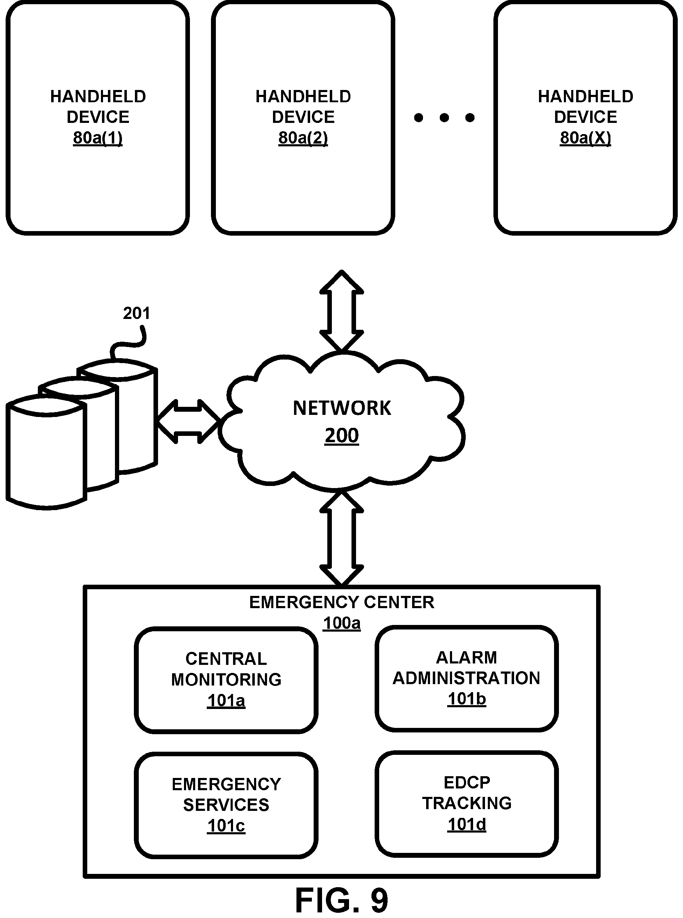

FIG. 9 illustrates an X number of handheld devices 80a, an emergency center 100a and a data storage 200, all connected to a network 200 via cloud application, Internet and GPS connectivity as known in the art.

Emergency center 100a provides a central monitoring 101a of external defibrillator chest pads of the present disclosure by connecting to associated handheld devices 80a. In practice, central monitoring 101a enables key diagnostics related information (e.g., pad impedance, degree of pad gel dryness, and issues with charging/discharging the external defibrillator chest pads).

Emergency center 100a further provides alarm administration 101b for local personnel associated with a patient. In practice, an external defibrillation chest pad of the present disclosure will communicate, to a handheld device 80a, event data and parameters related to the pad being used to rescue a patient (e.g., patient suffering from SCA/fibrillation) and in turn, upon emergency center 100a receiving said communication from handheld device 80a, alert administration 101b will create alarms for local caretakers (e.g., facility administrators, parents, guardians, etc.).

Emergency center 100a further provides emergency services 101c involving contacting professional responders (e.g., ambulances and hospitals). In practice, an external defibrillation chest pad of the present disclosure will communicate, to a handheld device 80a, the attempted rescue of a patient (e.g., patient suffering from SCA/fibrillation) and in turn, upon emergency center 100a receiving said communication from handheld device 80a, emergency services 101c will be activated to contact professional responder(s).

Emergency center 100a further provides external defibrillator chest pad (EDCP) tracking services 101d. In practice, a handheld device 80a will share a location of a patient with emergency center 100a when an associated external defibrillator chest pad is being used to rescue the patient whereby the location can be further shared with alert administration 101b and emergency service 101c.

Additional, emergency center 100a can be utilized to download upgrades of firmware, training scenarios, language updates, and related support to external defibrillator chest pads of the present disclosure via the cloud connection to handheld devices 80a.

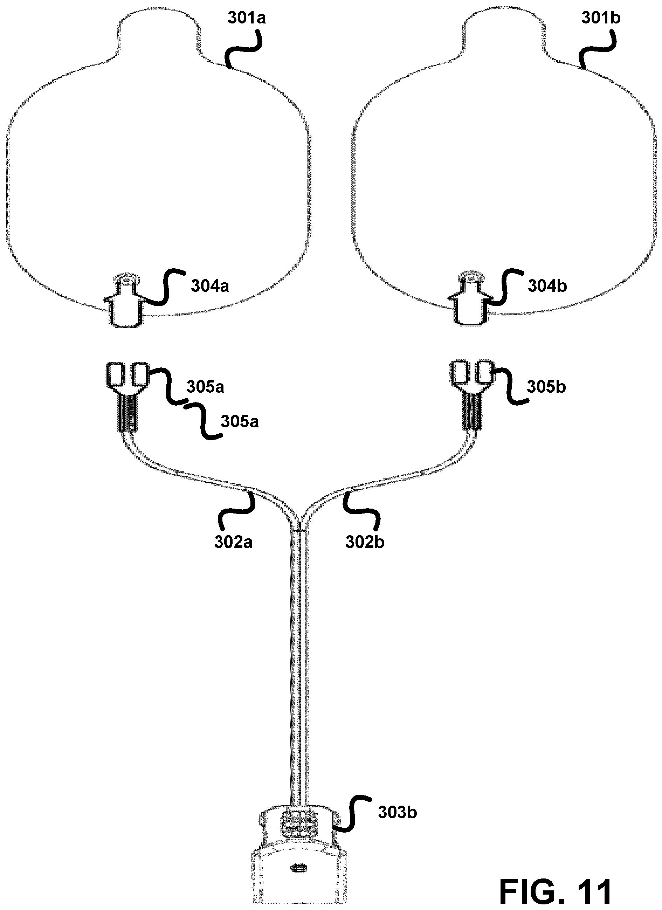

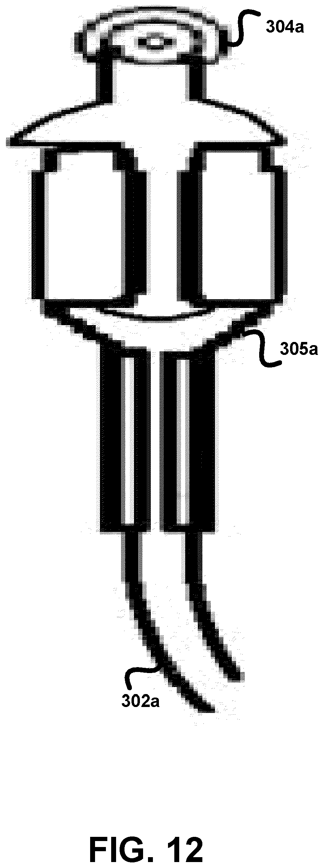

To facilitate a further understanding of the present disclosure, the following description of FIGS. 11 and 12 teaches exemplary embodiments of a spade connector in accordance with the present disclosure. From the description of FIGS. 11 and 12, those having ordinary skill in the art of the present disclosure will appreciate how to apply the present disclosure to make and use additional embodiments of spade connectors in accordance with the present disclosure.

Referring to FIG. 1, defibrillation electrode pads 21a and 21b as known in the art of the present disclosure are placed on a chest of patient 10 and connected to external defibrillator 20, whereby defibrillation electrode pad 21a and 21b allow external defibrillator 20 to analyze a rhythm of a heart of patient 10, and if necessary, defibrillation electrode pad 21a and 21b allow external defibrillator 20 to transmit an electric current to the heart of patient 10 that can restore a normal rhythm of the heart of patient 10.

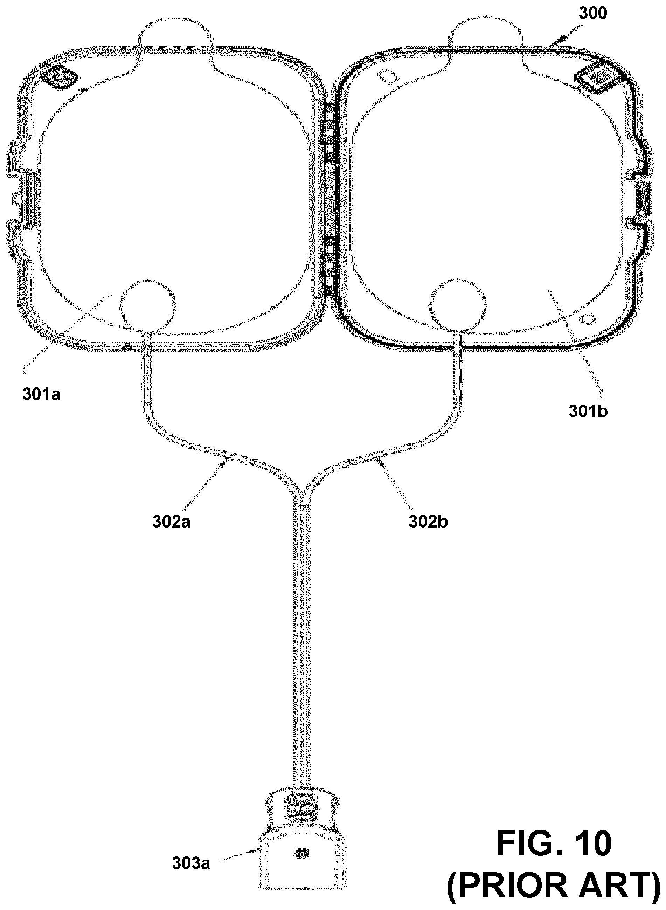

FIG. 10 illustrates a defibrillation pad assembly as known in the art of the present disclosure employing an electrode housing 300 for a sternum electrode pad 301 (e.g., defibrillation electrode pad 21a of FIG. 1) and an apex electrode pad 301b (e.g., defibrillation electrode pad 21b of FIG. 1).

Referring to FIG. 10, sternum electrode pad 301a has a permanent connection (i.e., non-detachable) via a wire 302a (e.g., a white wire) to a main connector 303 to an external defibrillator (not shown) and apex electrode paid 301b has a permanent connection (i.e., non-detachable) via a wire 302b (e.g., a blue wire) to the main connector 303a.

As known in the art of the present disclosure, once the defibrillation electrode pads 301a and 301b are placed on a patient's skin, defibrillation electrode pads 301a and 301b can be used to apply up to nine (9) shocks max. Thereafter, defibrillation electrode pads 301a and 301b must be disposed of.

Specifically, defibrillation electrode pads 301a and 301b are designed for a single use on a single patient. More particularly, when defibrillation electrode pads 301a and 301b have been applied on a patient, the gel on defibrillation electrode pads 301a and 301b eventually lose its stickiness and may not stick onto another patient's chest correctly. As such, the adhesive properties and the performance of the defibrillation electrode pads 301a and 301b will decay after the use, which makes them less effective, and defibrillation electrode pads 301a and 301b will not deliver the required shock therapy.

Also, once the seal of packaging pouch of defibrillation electrode pads 301a and 301b has been opened, the hydro gel of defibrillation electrode pads 301a and 301b will start to dry (with respect to temperature), which results in change in electrode impedance and further it will result in degradation in performance of the external defibrillator. Hence in this case also, the defibrillation pads must be disposed of.

Clearly, defibrillation electrode pads 301a and 301b are disposable objects and because of the permanent connections, wires 302a, 302b and main connector 303 also have to be disposed even wires 302a, 302b and main connector 303a are in good condition and working fine.

Referring to FIG. 11, the present disclosure provides a detachable connection of sternum electrode pad 301a to a wire 302a via a spade connector having a male spade socket 304a permanently attached to sternum electrode pad 301a and a female spade plug 305a permanently attached to wire 302a. The present disclosure provides a detachable connection of apex electrode pad 301b to a wire 302b via a spade connector having a male spade plug 304b permanently attached to sternum electrode pad 301b and a female spade plug 305b permanently attached to wire 302b.

Conversely (not shown), the present disclosure can provide a detachable connection of sternum electrode pad 301a to a wire 302a via a spade connector having female spade plug 305a permanently attached to sternum electrode pad 301a and a male spade plug 304a permanently attached to wire 302a. The present disclosure can further provide a detachable connection of apex electrode pad 301b to a wire 302b via a spade connector having female spade plug 305b permanently attached to sternum electrode pad 301b and male spade plug 304b permanently attached to wire 302b.

The opposite ends of lead wires 302a and 302b are attached to two (2) main connectors (one main connector 303b being shown), one pin for each lead wire, which in turn plugs into the external defibrillator.

In practice, a male spade plug and a female spade socket of the present disclosure are constructed facilitating a transmission of cardiac signals from a defibrillation electrode pad to an external defibrillator via a wire and a main connector, and facilitating a transmission of a defibrillation shock from the external defibrillator to a defibrillation electrode pad via a wire and the main connector.

In one exemplary embodiment, a male spade plug of the present disclosure (e.g., plugs 304a and305b) is made up of a tin coated thick copper metal. It's a metal elongation, whose one end is connected to tin electrode with the help of a rivet. Another end of this male spade plug has a flat rectangular tongue portion for mating with a female spade socket. The male spade plug is covered by foam to avoid the hazard of electrical shock to a user.

A female spade socket of the present disclosure (e.g., sockets 305a and 305b) is made up of tin-plated thick brass. Its back end connected or crimped permanently to a pre-stripped end of a pads wire. Front end of this female spade socket has a tongue portion with hole which grip a male spade plug in a separable, reusable manner, forming a quick connect wiring termination. The female spade socket is vinyl or PVC insulated to avoid the hazard of electrical shock to the user. The opposite ends of lead wires are attached to two main connectors, one pin for each lead wire, which in turn plugs into the AED.

As will be appreciated by those having ordinary skill in the art, the exemplary construction/structure of a spade connector of the present disclosure provides electrical continuity from a defibrillation electrode pad through the spade connector to a lead wire, which is an enhancement. Further, construction/structure of a spade connector of the present disclosure will help with quick reuse of defibrillation electrode pads (i.e. quick disconnect and quick connect of wiring connection).

Still referring to FIG. 11, prior to a first use, male spade plugs 304 (connected to defibrillation electrode pads 301) will be pushed into a female spade sockets 305 (connected to wires 302 and main connector assembly 303b) as shown in FIG. 12, whereby wires 302 and main connector assembly 303b become fully connected to defibrillation electrode pad 301.

The main connector assembly 303b is thereafter connected to the external defibrillator (e.g., external defibrillator 20 of FIG. 2) and defibrillation electrode pads 301 are adhered to the patient's chest on the patient's chest.

After delivery of the required shock(s) or at the time of replacing defibrillation electrode pads 301, main connector assembly 303b will first be disconnected from the external defibrillator, so that defibrillation electrode pads 301 are fully detached from the external defibrillator. Thereafter, the male spade plugs 304 are pulled from the respective female spade sockets 305 whereby the wires 302 and main connector assembly 303 are fully isolated from defibrillation electrode pads 301, which can now be disposed of.

The present disclosure provides for a reuse of the same (existing) wires 302 and main connector assembly 303b by connecting new (unused) defibrillation electrode pads to wires 302 and main connector assembly 303b. Specifically, new male spade plugs 304 connected to the new defibrillation electrode pads 301 are pushed into respective female spade sockets 305 whereby wires 302 and main connector assembly 303b are fully connected to new (unused) defibrillation electrode pads, which are now ready for use with the external defibrillator.

The same process of using the present disclosure applies when the female spade sockets are permanently attached to defibrillation electrode pads, and the male spade plugs are attached to the wires.

In additional embodiments of the present disclosure, other connector types having a plug and socket can be used in place of a spade connector of the present disclosure.

Further, wires 302 can be radiotransparent whereby the defibrillation electrode pads can be used in image analysis (e.g., X-rays or computed-tomography).

In one exemplary embodiment, a spade connector of the present disclosure can be used to connect defibrillation electrodes 70a and 70b as shown in FIG. 6 to various components within an external defibrillation chest pads of the present disclosure whereby conductive pads 45a and 45b can be replaced as needed without having to dispose of the external defibrillation chest pads when conductive pads 45a and 45b are no longer usable.

From the description of FIGS. 2-9, 11 and 12 herein, those having ordinary skill in the art will appreciate the numerous benefits of the present disclosure including, but not limited to, (1) an accessible and easy to use external defibrillator design with an ability to detect heart arrythmia & provide a high energy shock to the heart of a person facing a Sudden Cardiac Arrest (SCA), and (2) an easy plug and play method to connect new electrode with existing wires and connector.

The present disclosure has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

Further, as one having ordinary skill in the art shall appreciate in view of the teachings provided herein, features, elements, components, etc. disclosed and described in the present disclosure/specification and/or depicted in the appended Figures and/or recited in the Claims can be implemented in various combinations of hardware and software, and provide functions which may be combined in a single element or multiple elements. For example, the functions of the various features, elements, components, etc. shown/illustrated/depicted in the Figures and/or recited in the Claims can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and exemplary embodiments of the present disclosure, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar functionality, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the present disclosure, which embodiments are intended to be illustrative and not limiting, it is noted that modifications and variations can be made by persons having ordinary skill in the art in view of the teachings provided herein, including the appended Figures and claims. It is therefore to be understood that changes can be made in/to the preferred and exemplary embodiments of the present disclosure which are within the scope of the present disclosure and exemplary embodiments disclosed, described and taught herein.

Moreover, it is contemplated that corresponding and/or related systems incorporating and/or implementing the device or such as may be used/implemented in a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure.

## Claims

1. An external defibrillation chest pad (40), comprising:
the external defibrillation chest pad housing a defibrillation controller (50), a defibrillation shock module (60) and a pair of defibrillation electrode pads (70); and
wherein, when the external defibrillation chest pad (40) is adhered to a chest of a patient, when the external defibrillation chest pad (40) is in data communication with a hand-held device (80), and when the external defibrillation chest pad (40) is in power communication with at least one of the hand-held device (80) and a power bank (90),
the pair of defibrillation electrode pads (70) are configured to conduct electrical cardiac signals of the patient to the defibrillation controller (50),
the defibrillation controller (50) is configured to analyze an electrocardiogram of the patient derived from the electrical cardiac signals conducted by the pair of defibrillation electrode pads (70) to the defibrillation controller (50) and to communicate the electrocardiogram of the patient by the defibrillation controller (50) to the hand-held device (80), and
the defibrillation controller (50) is further configured to control a cardiac therapy from the defibrillation shock module (60) through the pair of defibrillation electrode pads (70) to the patient based on the analysis of the electrocardiogram of the patient by the defibrillation controller (50) indicating a shockable rhythm within the electrocardiogram of the patient.

2. The external defibrillation chest pad (40) of claim 1,
where the analysis of the electrocardiogram of the patient by the defibrillation controller (50) includes the defibrillation controller (50) configured to execute a shock advisory derived from the electrocardiogram of the patient; and
wherein the control of the cardiac therapy by the defibrillation controller (50) includes the defibrillation controller (50) configured to automatically execute a shock delivery from the defibrillation shock module (60) through the pair of defibrillation electrode pads (70) to the patient when the shock advisory renders a shock decision derived from the shockable rhythm within the electrocardiogram of the patient.

3. The external defibrillation chest pad (40) of claim 1,
wherein the analysis of the electrocardiogram of the patient by the defibrillation controller (50) includes the defibrillation controller (50) configured to execute a shock advisory of the electrocardiogram of the patient;
wherein, when the shock advisory renders a shock decision, the defibrillation controller (50) is configured to communicate the shock decision to the hand-held device (80); and
wherein the control of the cardiac therapy by the defibrillation controller (50) includes the defibrillation controller (50) configured to execute a shock delivery from the defibrillation shock module (60) through the pair of defibrillation electrode pads (70) to the patient when the hand-held device (80) communicates a shock activation signal to the defibrillation controller (50) subsequent to the communication of the shock decision from the defibrillation controller (50) to the hand-held device (80).

4. The external defibrillation chest pad (40) of claim 1,
wherein a conditional execution of the cardiac therapy by the defibrillation controller (50) includes the defibrillation controller (50) configured to execute a shock delivery from the defibrillation shock module (60) through the pair of defibrillation electrode pads (70) to the patient when the hand-held device (80) communicates a shock activation signal to the defibrillation controller (50) subsequent to the communication of the electrocardiogram from the defibrillation controller (50) to the hand-held device (80).

5. The external defibrillation chest pad (40) of claim 1,
wherein, when the defibrillation controller (50) receives a communication by the hand-held device (80) of a cardiopulmonary resuscitation of the patient, the analysis of the electrocardiogram of the patient by the defibrillation controller (50) includes the defibrillation controller (50) configured to execute a shock advisory of the electrocardiogram of the patient based on the cardiopulmonary resuscitation of the patient.

6. The external defibrillation chest pad (40) of claim 1,
wherein the defibrillation controller (50) is configured to control a charging of the defibrillation shock module (60) by the at least one of the hand-held device (80) and the power bank (90).

7. The external defibrillation chest pad (40) of claim 1,
wherein each defibrillation electrode pads (70) of the pair of defibrillation electrode pads (70) is detachably connected from the external defibrillation chest pad (40) by a spade connector.

8. An external defibrillation system, comprising:
a hand-held device (80); and
an external defibrillation chest pad housing a defibrillation controller (50), a defibrillation shock module (60) and a pair of defibrillation electrode pads (70); and
wherein, when the external defibrillation chest pad (40) is adhered to a chest of a patient, when the external defibrillation chest pad (40) is in data communication with a hand-held device (80), and when the external defibrillation chest pad (40) is in power communication with at least one of the hand-held device (80) and a power bank (90),
the pair of defibrillation electrode pads (70) are configured to conduct electrical cardiac signals of the patient to the defibrillation controller (50),
the defibrillation controller (50) is configured to analyze an electrocardiogram of the patient derived from the electrical cardiac signals conducted by the pair of defibrillation electrode pads (70) to the defibrillation controller (50) and to communicate the electrocardiogram of the patient by the defibrillation controller (50) to the hand-held device (80), and
the defibrillation controller (50) is further configured to control a cardiac therapy from the defibrillation shock module (60) through the pair of defibrillation electrode pads (70) to the patient based on the analysis of the electrocardiogram of the patient by the defibrillation controller (50) indicating a shockable rhythm within the electrocardiogram of the patient.

9. The external defibrillation system of claim 8,
where the analysis of the electrocardiogram of the patient by the defibrillation controller (50) includes the defibrillation controller (50) configured to execute a shock advisory derived from the electrocardiogram of the patient; and
wherein the control of the cardiac therapy by the defibrillation controller (50) includes the defibrillation controller (50) configured to automatically execute a shock delivery from the defibrillation shock module (60) through the pair of defibrillation electrode pads (70) to the patient when the shock advisory renders a shock decision derived from the shockable rhythm within the electrocardiogram of the patient.

10. The external defibrillation system of claim 8,
wherein the analysis of the electrocardiogram of the patient by the defibrillation controller (50) includes the defibrillation controller (50) configured to execute a shock advisory of the electrocardiogram of the patient;
wherein, when the shock advisory renders a shock decision, the defibrillation controller (50) is configured to communicate the shock decision to the hand-held device (80); and
wherein the control of the cardiac therapy by the defibrillation controller (50) includes the defibrillation controller (50) configured to execute a shock delivery from the defibrillation shock module (60) through the pair of defibrillation electrode pads (70) to the patient when the hand-held device (80) communicates a shock activation signal to the defibrillation controller (50) subsequent to the communication of the shock decision from the defibrillation controller (50) to the hand-held device (80).

11. The external defibrillation system of claim 8,
wherein a conditional execution of the cardiac therapy by the defibrillation controller (50) includes the defibrillation controller (50) configured to execute a shock delivery from the defibrillation shock module (60) through the pair of defibrillation electrode pads (70) to the patient when the hand-held device (80) communicates a shock activation signal to the defibrillation controller (50) subsequent to the communication of the electrocardiogram from the defibrillation controller (50) to the hand-held device (80).

12. The external defibrillation system of claim 8,
wherein, when the defibrillation controller (50) receives a communication by the hand-held device (80) of a cardiopulmonary resuscitation of the patient, the analysis of the electrocardiogram of the patient by the defibrillation controller (50) includes the defibrillation controller (50) configured to execute a shock advisory of the electrocardiogram of the patient based on the cardiopulmonary resuscitation of the patient.

13. The external defibrillation system of claim 8,
wherein the defibrillation controller (50) is configured to control a charging of the defibrillation shock module (60) by at least one of the hand-held device (80) and the power bank (90).

14. The external defibrillation system of claim 8,
wherein each defibrillation electrode pads (70) of the pair of defibrillation electrode pads (70) is detachably connected from the external defibrillation chest pad (40) by a spade connector.

15. An external defibrillation method executable by an external defibrillation chest pad (40), housing a defibrillation controller (50), a defibrillation shock module (60) and a pair of defibrillation electrode pads (70),
the external defibrillation method comprising:
a pre-operation phase including:
adhering the external defibrillation chest pad (40) to a chest of a patient,
establishing data communication between the external defibrillation chest pad (40) and a hand-held device (80), and
establishing a power communication between the external defibrillation chest pad (40) at least one of the hand-held device (80) and a power bank (90);
an operation phase including:
conducting, by the pair of defibrillation electrode pads (70), electrical cardiac signals of the patient to the defibrillation controller (50),
analyzing, by the defibrillation controller (50), an electrocardiogram of the patient derived from the electrical cardiac signals conducted by the pair of defibrillation electrode pads (70) to the defibrillation controller (50),
communicating, by the defibrillation controller (50), the electrocardiogram of the patient to the hand-held device (80), and
controlling, by the defibrillation controller (50) a cardiac therapy from the defibrillation shock module (60) through the pair of defibrillation electrode pads (70) to the patient based on the analysis of the electrocardiogram of the patient by the defibrillation controller (50) indicating a shockable rhythm within the electrocardiogram of the patient.
